# FASCICULE DE BREVET EUROPEEN

(11) **EP 3 687 459 B1**
(45) Date de publication et mention de la délivrance du brevet: **04.08.2021**
(21) Numéro de dépôt: 18785855.0
(22) Date de dépôt: 27.09.2018
(51) Int. Cl.: A61F 2/46, A61B 17/92

(54) **DISPOSITIF D'INSERTION D'UN IMPLANT CHIRURGICAL**
VORRICHTUNG ZUM EINSETZEN EINES CHIRURGISCHEN IMPLANTATS
DEVICE FOR INSERTING A SURGICAL IMPLANT

(30) Priorité: 29.09.2017 FR 1759130
(43) Date de publication de la demande: 05.08.2020
(73) Titulaire: Centre National de la Recherche Scientifique, 75016 Paris (FR); Université Paris XII Val de Marne, 94010 Créteil Cedex (FR)
(72) Inventeur: HAIAT, Guillaume, 94150 Rungis (FR); ROSI, Giuseppe, 75013 Paris (FR); TIJOU, Antoine, 69004 Lyon (FR)
(74) Mandataire: Osha Liang
(86) Numéro de dépôt international: PCT/EP2018/076228
(87) Numéro de publication internationale: WO 2019/063675

(56) Documents cités:
- EP-A1- 2 923 677
- US-A1- 2015 282 856
- US-A1- 2017 196 711

## Description

### DOMAINE TECHNIQUE

L'invention concerne un dispositif pour insérer un implant chirurgical par impaction dans un os récepteur. Ce dispositif comprend un outil de percussion, ou impacteur, adapté pour exercer une force d'impaction sur l'implant chirurgical afin d'insérer en force celui-ci dans l'os récepteur.

### ARRIERE PLAN

Dans le présent exposé, la notion d'implant chirurgical ne se limite pas aux implants chirurgicaux proprement dits mais englobe également les instruments chirurgicaux insérés temporairement dans un os récepteur. Cette notion recouvre, en particulier, l'ensemble des implants de prothèse orthopédique destinés à remplacer une articulation déficiente, notamment les implants de prothèses de hanche, de genoux, d'épaule, de rachis, de coude ou de cheville, et les instruments de pose utilisés pour préparer l'os récepteur à recevoir la prothèse.

L'invention se rapporte plus particulièrement, mais non exclusivement, à la mise en place d'une prothèse de hanche. La grande majorité des prothèses de hanche ont en commun une première partie fixée sur le fémur et une deuxième partie fixée sur le bassin. La première partie comprend une tige fémorale destinée à être insérée en force dans le canal médullaire du fémur et une tête prothétique formée d'une pièce sphérique, montée sur la tige fémorale et se substituant à la tête du fémur. La seconde partie comprend une cotyle prothétique destiné à être inséré dans la cavité cotyloïdienne située sur la face latérale de l'os iliaque du bassin pour remplacer la partie articulaire du bassin. La cotyle prothétique peut comprendre une cupule cotyloïdienne, qui est une pièce approximativement hémisphérique, généralement en métal, insérée dans la cavité cotyloïdienne et dans laquelle se place un insert avec lequel s'articule la tête prothétique.

L'insertion de la tige fémorale dans le canal médullaire est généralement réalisée par impaction au moyen d'un impacteur, typiquement un marteau. Un ancillaire de pose peut également être utilisé, le praticien utilisant l'impacteur pour frapper l'ancillaire qui transmet la force d'impaction à la tige fémorale.

Au fur et à mesure des impacts, la tige s'enfonce dans le canal médullaire. La tige s'enfonçant sur une grande partie de sa longueur, le déplacement relatif entre la tige et le fémur est important. Le niveau de contact entre la tige et le fémur qui l'entoure dépend de cet enfoncement. Ce niveau de contact est généralement caractérisé par le ratio BIC (en anglais "BIC ratio" pour "bone to implant contact ratio") qui est le pourcentage de la surface de l'implant au contact de l'os. Plus la tige s'enfonce, plus la surface de la tige au contact du fémur augmente.

Le praticien souhaite avant tout suivre l'enfoncement de la tige dans le fémur et déterminer le moment où le niveau de contact entre la tige et le fémur est optimal ou, tout au moins, satisfaisant. La réussite de l'opération repose à la fois sur un niveau de contact suffisant entre la tige et le fémur et sur le fait de ne pas endommager et notamment de ne pas induire de fracture ou de microfissures dans le fémur lors de l'insertion. Si la tige fémorale est insuffisamment insérée dans le fémur, il peut s'ensuivre des micromouvements de la tige pouvant nécessiter une nouvelle intervention chirurgicale.

Un compromis doit donc être trouvé entre un nombre d'impacts suffisamment élevé pour obtenir un niveau de contact satisfaisant entre la tige et le fémur et suffisamment faible pour ne pas risquer d'endommager le fémur. Or, il est difficile pour le praticien d'évaluer par lui-même avec fiabilité le bon nombre d'impacts. Concrètement, il lui est difficile de savoir précisément quand arrêter de frapper la tige fémorale avec l'impacteur.

Dans ce contexte et, plus généralement, dans le contexte de l'insertion en force d'un implant chirurgical dans un os récepteur par impaction, un but de l'invention est de proposer un dispositif permettant de fournir, durant l'opération chirurgicale, une information fiable sur le niveau de contact entre l'implant et l'os récepteur et de permettre ainsi au praticien de savoir, en temps réel, quand il doit arrêter de frapper l'implant avec l'impacteur.

Le document de brevet FR 3019031 décrit une technique d'assistance à la mise en place d'un implant orthopédique, dans laquelle est calculé un indicateur corrélé à la force d'arrachement de l'implant et reflétant la stabilité implantaire. Cet indicateur est très utile dans de nombreuses applications, en particulier pour la mise en place d'un cotyle prothétique dans la cavité cotyloïdienne. Toutefois, dans des applications comme l'insertion de tige fémorale, d'autres indicateurs pourraient être plus utiles, ou tout au moins aussi utiles au praticien. L'utilisation de plusieurs indicateurs pourrait, par ailleurs, s'avérer intéressante dans certaines applications.

Le document US2015/282856 A1 décrit un dispositif selon le préambule de la revendication 1.

### PRESENTATION GENERALE

L'invention concerne un dispositif pour insérer en force un implant chirurgical dans un os récepteur, par impaction. Ce dispositif comprend un impacteur adapté pour impacter une surface d'impact couplée audit implant chirurgical et exercer une force d'impaction sur l'implant.

L'impacteur est associé à au moins un capteur apte à mesurer une grandeur parmi la force d'impaction exercée et la déformation de l'impacteur, et à fournir un signal de mesure représentant la variation temporelle de ladite grandeur lors d'un impact.

Le capteur (i.e. ledit au moins un capteur) est relié à une unité de traitement configurée pour déterminer à partir de la variation temporelle de ladite grandeur lors de l'impact, un indicateur représentatif du niveau de contact entre l'implant et l'os récepteur. La liaison électronique entre le capteur et l'unité de traitement peut être filaire ou non.

La solution proposée repose sur la mise en œuvre d'un ou plusieurs capteurs associés à l'impacteur et délivrant un signal de mesure, l'enregistrement et l'analyse de ce signal permettant de déterminer un indicateur révélateur du niveau de contact entre l'implant et l'os récepteur. Lorsque plusieurs capteurs sont utilisés, les signaux respectivement délivrés par ces capteurs peuvent être, par exemple, moyennés ou combinés pour obtenir le signal de mesure qui sera analysé et à partir duquel l'indicateur sera calculé.

Un tel dispositif permet, au cours de l'opération d'insertion de l'implant chirurgical, de renseigner en temps réel le praticien sur le niveau de contact atteint entre l'implant et l'os récepteur. Outre son coût réduit, ce dispositif a l'avantage d'être simple d'utilisation. En particulier, avec ce dispositif, le geste du praticien lors de l'opération reste le même. Le praticien n'a donc pas à apprendre de nouveaux gestes et peut tirer profit de l'expérience qu'il a déjà acquise avec les dispositifs conventionnels.

L'indicateur proposé correspond à la durée d'une fenêtre temporelle, le début de cette fenêtre temporelle étant défini par rapport à un instant correspondant au premier pic d'amplitude maximale du signal de mesure et la fin de cette fenêtre temporelle étant définie par rapport à un instant correspondant au deuxième pic d'amplitude maximale du signal de mesure. Il a été montré que l'indicateur ainsi calculé était corrélé au niveau de contact entre l'implant et l'os récepteur et constituait un indicateur fiable.

Dans certains modes de réalisation le dispositif comprend, en outre, un système d'alerte relié à l'unité de traitement et coopérant avec celle-ci de manière à émettre un signal d'alerte lorsque l'indicateur devient inférieur à (i.e. descend en dessous de) une valeur seuil prédéterminée. Cette valeur seuil peut être déterminée expérimentalement. Par exemple, on réalise des tests et on choisit comme valeur seuil la valeur de l'indicateur à partir de laquelle on constate un niveau de contact suffisant entre l'implant et l'os récepteur. En particulier, la valeur seuil peut être comprise entre 0,1 et 1 ms.

Ainsi, le fait que la durée de ladite fenêtre temporelle devienne inférieure à une certaine durée, prédéterminée, est utilisé comme condition pour émettre le signal d'alerte (e.g. une lumière, un son, une vibration, etc.). Le praticien, averti par ce signal, sait alors qu'il doit arrêter d'impacter l'implant, le niveau de contact entre l'implant et l'os étant considéré comme optimal ou, en tous cas, comme suffisant.

Bien entendu, d'autres conditions relatives à l'indicateur lui-même ou à la variation de l'indicateur lors d'une série d'impacts successifs pourraient être utilisées pour déclencher une alerte, sans sortir du cadre de l'invention. En particulier, il est possible de tirer profit du fait que, lorsqu'un niveau de contact os-implant satisfaisant est atteint, l'indicateur proposé tend à se stabiliser (i.e. converge vers une valeur stationnaire).

Dans certains modes de réalisation, l'unité de traitement détecte dans le signal de mesure le premier pic d'amplitude maximale et le pic d'amplitude maximale succédant au premier pic, ce dernier pic n'étant considéré comme le deuxième pic d'amplitude maximale que si le signal de mesure devient inférieur à une valeur limite prédéterminée entre ces deux pics (i.e. si le signal de mesure descend au-dessous de la valeur limite avant de repasser au-dessus de cette valeur pour former le deuxième pic). En particulier, la valeur limite peut être comprise entre 1 à 20% de l'amplitude maximale du premier pic. Par exemple, le pic d'amplitude maximale succédant au premier pic n'est considéré comme le deuxième pic d'amplitude maximale que si le signal de mesure passe au-dessous d'une valeur limite égale à 5% de l'amplitude maximale du premier pic.

Cette précaution permet d'éviter les erreurs de mesure liées à un phénomène de dédoublement du premier pic, qui a pu être observé dans un petit nombre de cas. Lors d'un tel phénomène, les deux pics obtenus par dédoublement du premier pic sont proches l'un de l'autre et les inventeurs ont réalisé que le signal de mesure n'avait pas le temps de diminuer significativement entre ces deux pics. Aussi, la solution consistant à vérifier que le signal de mesure a suffisamment diminué avant d'atteindre le deuxième pic d'amplitude maximale, permet d'éviter de considérer à tort le doublon du premier pic comme le deuxième pic d'amplitude maximale, et donc d'éviter une erreur de mesure sur l'indicateur. Bien entendu, d'autres méthodes d'analyse du signal de mesure pourraient être envisagées pour détecter un dédoublement du premier pic et éviter des erreurs de mesure dans un tel cas.

Dans certains modes de réalisation, l'impacteur a une face de frappe adaptée pour impacter la surface d'impact et le capteur est un capteur de force apte à mesurer la force d'impaction et à fournir un signal de mesure représentant la variation temporelle de la force d'impaction lors d'un impact.

Dans d'autres modes de réalisation, l'impacteur a une face de frappe adaptée pour impacter la surface d'impact, une face opposée à la face de frappe et des faces latérales s'étendant entre la face de frappe et la face opposée, et le capteur est un capteur de déformation apte à mesurer la déformation de l'impacteur et à fournir un signal de mesure représentant la variation temporelle de la déformation de l'impacteur lors d'un impact.

Dans certains modes de réalisation, l'impacteur est un marteau, ou équivalent, et comprend un manche surmonté d'une tête de frappe. En particulier, l'impacteur peut avoir sensiblement la même forme et le même poids que les impacteurs couramment utilisés jusqu'à présent. Ainsi, les praticiens expérimentés sont immédiatement en mesure de manier correctement l'impacteur.

On notera que la surface d'impact peut-être couplée directement à l'implant en ce sens qu'il peut s'agir d'une des surfaces de l'implant, ou peut être indirectement couplée à l'implant en ce sens qu'il peut s'agir d'une surface d'un instrument, ou ancillaire de pose, venant lui-même au contact de l'implant. Dans ce dernier cas, l'impacteur exerce la force d'impaction sur l'implant par l'intermédiaire de l'ancillaire de pose. En d'autres termes, la force d'impaction est exercée sur l'ancillaire de pose et transmise par celui-ci à l'implant.

Dans certains modes de réalisation, le dispositif comprend un ancillaire de pose ayant une extrémité arrière formant ladite surface d'impact et une extrémité avant adaptée pour coopérer avec l'implant, l'impacteur exerçant la force d'impaction sur l'implant par l'intermédiaire de l'ancillaire de pose.

L'extrémité avant de l'ancillaire de pose peut coopérer avec l'implant par simple contact. En variante, l'extrémité avant de l'ancillaire de pose peut être attachée mécaniquement à l'implant orthopédique de manière amovible, par exemple par vissage. Le fait d'attacher l'ancillaire à l'implant permet, généralement, d'obtenir un meilleur signal de mesure. L'ancillaire est amovible de manière à pouvoir détacher facilement l'ancillaire de l'implant une fois ce dernier en position.

Le présent exposé concerne également un ensemble comprenant un dispositif tel que précédemment décrit et un implant chirurgical, en particulier une tige fémorale.

La présente description décrit également un procédé n'appartenant pas à l'invention pour insérer en force un implant chirurgical dans un os récepteur, par impaction, dans lequel:
- on fournit un dispositif tel que précédemment décrit,
- on exerce avec l'impacteur une force d'impaction sur l'implant, en impactant une surface d'impact couplée à l'implant, de manière à insérer l'implant,
- on calcule l'indicateur lors d'impacts successifs, et
- on arrête d'impacter la surface d'impact lorsque l'indicateur devient inférieur à une valeur seuil prédéterminée.

L'implant chirurgical peut être, mais n'est pas nécessairement, une tige fémorale. Dans ce cas, on impacte la tige fémorale avec l'impacteur de manière à insérer en force la tige dans le canal médullaire du fémur d'un patient.

Les avantages d'un tel procédé découlent des avantages du dispositif utilisé.

Les caractéristiques et avantages précités, ainsi que d'autres, apparaîtront à la lecture de la description détaillée qui suit, d'exemples de réalisation du dispositif proposé. Cette description détaillée fait référence aux dessins annexés.

### BREVE DESCRIPTION DES DESSINS

Les dessins annexés sont schématiques et ne sont pas à l'échelle, ils visent avant tout à illustrer les principes de l'invention.
- la figure 1 est une représentation schématique d'un dispositif pour insérer un implant chirurgical, comprenant un impacteur muni d'un capteur de force.
- la figure 2 représente schématiquement un exemple de signaux obtenus à l'aide du capteur de force de la figure 1, lors de l'insertion de l'implant.
- la figure 3 représente schématiquement la variation de l'indicateur retenu et la variation de l'enfoncement de l'implant en fonction du nombre d'impacts.

La figure (FIG) 1 représente un exemple de dispositif 1 pour insérer progressivement un implant chirurgical dans un os récepteur, par impaction. Dans cet exemple, l'implant est un implant pour prothèse, en particulier une tige fémorale 2 pour prothèse de hanche. Cette tige 2 est destinée à être insérée progressivement par impaction dans le canal médullaire 3 du fémur 4 d'un patient. Le canal médullaire 3 est, généralement, préalablement préparé par le praticien pour recevoir la tige 2. Dans tous les cas, les dimensions de la tige 2 sont légèrement supérieures aux dimensions du canal médullaire 3 et la tige 2 est insérée en force dans ce canal.

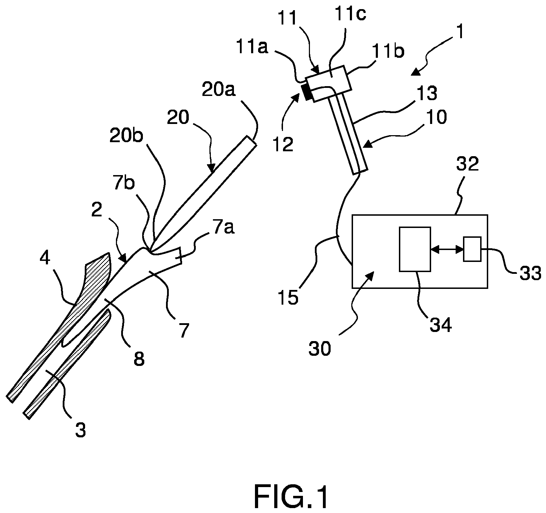

La tige fémorale 2 comprend un col prothétique 7 destiné à dépasser du fémur 4 et dont l'extrémité 7a reçoit une sphère (non représentée), et un corps 8 effilé s'étendant à partir du col 7 et dont la section transversale diminue à mesure que l'on s'éloigne du col 7. Le corps 8 est destiné à être enfoncé intégralement dans le fémur 4. Sur la FIG 1, le corps 8 est représenté partiellement enfoncé dans le fémur 4. Le col 7 présente également, à côté de son extrémité 7a, une surface d'appui 7b pour un ancillaire de pose 20.

Dans cet exemple, l'ancillaire de pose 20 est une barre ayant une extrémité arrière formant une surface d'impact 20a et une extrémité avant 20b adaptée pour venir au contact de la surface d'appui 7b de la tige 2. L'extrémité avant 20b de l'ancillaire 20 peut coopérer avec la tige 2 par simple contact avec la surface d'appui 7b ou être attachée mécaniquement à la tige de manière amovible, par exemple par vissage. Dans ce dernier cas, l'extrémité avant 20b de l'ancillaire 20 peut être filetée pour être vissée dans un trou taraudé (non représenté) ménagé dans la surface de contact 7b. L'avant et l'arrière sont définis ici par rapport à la direction d'avancement de la tige 2 et de l'ancillaire 20 lors de l'impaction.

Le dispositif 1 comprend également un outil de percussion ou impacteur 10, de type marteau ou équivalent, comprenant un manche 13 surmonté d'une tête de frappe 11. La tête de frappe 11 a une face de frappe 11a adaptée pour impacter la surface d'impact 20a de l'ancillaire 20, une face 11b opposée à la face de frappe 11a et des faces latérales 11c s'étendant entre la face de frappe 11a et la face opposée 11b. Lorsqu'il souhaite enfoncer la tige fémorale 2 dans le fémur 4, le praticien saisit l'ancillaire de pose 20 d'une main et le manche 13 de l'impacteur 10 de l'autre main. Il frappe ensuite la surface d'impact 20a de l'ancillaire 20 avec la face de frappe 11a de l'impacteur. La force d'impaction générée par l'impacteur 10 est transmise à la tige fémorale 2 par l'intermédiaire de l'ancillaire 20.

Dans l'exemple de la FIG 1, l'impacteur 10 est équipé d'un capteur de force 12 apte à mesurer la force d'impaction exercée sur l'implant par l'impacteur 10 et à fournir un signal de mesure représentant la variation temporelle de la force d'impaction lors d'un impact. Un exemple de capteur de force de ce type est décrit dans le document de brevet FR 3019031. Dans une situation d'insertion d'une tige fémorale 2 comme celle représentée sur la FIG 1, un tel capteur de force 12 est apte à convertir en un signal électrique exploitable la force d'impaction appliquée sur la surface d'impact 20a de l'ancillaire de pose 20 lors de chaque frappe. Il s'agit, par exemple, d'un capteur à jauge ou d'un capteur piézo-électrique connecté selon un montage approprié à l'unité de traitement 30. Dans l'exemple représenté, le capteur de force 12 est fixée sur la face de frappe 11a de la tête 11 de l'impacteur 10. En variante, le capteur de force 12 peut être positionné au niveau de la surface d'impact 20a de l'ancillaire de pose 20, voir au niveau de l'interface entre l'ancillaire de pose 20 et la tige fémorale. Dans les applications n'utilisant pas d'ancillaire, le capteur de force 12 peut être positionné au niveau de la surface de l'implant 2 qui forme la surface d'impact.

Le dispositif comprend également une unité de traitement 30 couplée au capteur 12 et configurée pour quantifier le contact entre la tige 2 et l'os récepteur, i.e. le fémur 4, à partir des signaux de mesure délivrés par le capteur 12. Cette unité de traitement 30 comprend, par exemple, un microcontrôleur 34. L'unité de traitement 30 peut être logée dans un boîtier externe 32. En variante, l'unité de traitement 30 peut être intégrée à l'impacteur 10. Selon une autre variante, l'unité de traitement 30 peut être formée d'éléments séparés comme un micro-ordinateur relié à un module d'acquisition de données lui-même relié au capteur 12.

La liaison entre le capteur 12 et l'unité de traitement 30 est, dans l'exemple de la FIG 1, réalisée de manière filaire au moyen d'un câble 15. En variante, la transmission des signaux de mesure fournis par le capteur 12 peut se faire au moyen d'une liaison sans fil, auquel cas le capteur 12 est équipé d'une antenne ou équivalent.

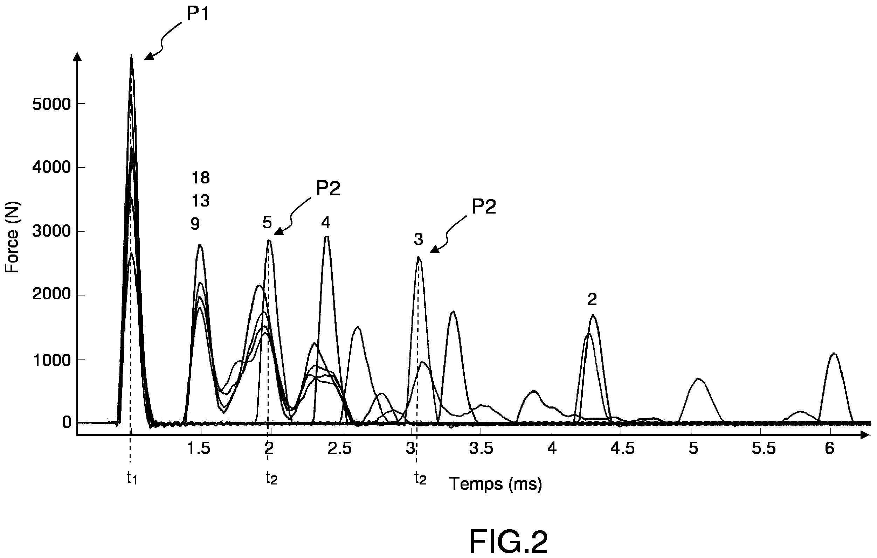

Lors de chaque impact effectué par le praticien sur la tige 2 au moyen de l'impacteur 10, via l'ancillaire de pose 20, le capteur 12 mesure la force d'impaction exercée et fournit un signal de mesure représentant la variation temporelle de cette force pendant l'impact. On considère que l'impact commence à partir de l'instant où l'impacteur 10 et l'implant entrent en contact, directement ou indirectement (i.e. via l'ancillaire 20), et dure pendant un certain laps de temps après cet instant. En tout état de cause, ce laps de temps est inférieur à 50 ms. Des exemples de signaux fournis par le capteur 12 sont représentés sur la FIG. 2 et décrits ci-après.

Les inventeurs ont eu l'idée de s'intéresser à un tel signal de mesure et ont mis en évidence le fait que ce signal portait des informations sur le niveau de contact entre le fémur 4 et la tige 2. En particulier, les inventeurs ont réussi à déterminer, à partir du signal de mesure recueilli, un indicateur représentatif du niveau de contact entre le fémur 4 et la tige 2, comme expliqué ci-après.

Pour essayer d'expliquer le lien entre le signal de mesure recueilli et le niveau de contact tige-fémur, l'explication suivante peut être avancée. L'impacteur 10 exerce sur la tige 2, via l'ancillaire 20, une force d'impaction qui est à l'origine de modes de vibration dans l'ensemble du système formé par l'impacteur 10, le capteur 12, l'ancillaire 20, la tige fémorale 2 et le fémur 4 lorsque ces éléments sont tous en contact lors de l'impact. Ces modes de vibration dépendent essentiellement des modes de vibration du système os-implant (i.e. du système fémur-tige) qui dépendent à leur tour du niveau de contact entre l'implant et l'os. Schématiquement, plus le niveau de contact os-implant est élevé, plus le système os-implant est rigide et plus les fréquences de résonnance des modes de vibration sont élevées.

La FIG 2 est un graphique représentant schématiquement un exemple de signaux obtenus à l'aide du capteur de force 12 lors d'un test d'insertion d'une tige fémorale 2 dans le fémur 4 d'un cadavre. Le temps (t) en millisecondes (ms) est reporté en abscisse, la force mesurée par le capteur 12, en Newtons (N), est reportée en ordonnée.

Plusieurs signaux sont représentés sur la FIG 2. Chaque signal correspond à un impact. Au total, lors de ce test d'insertion, vingt cinq impacts ont été portés sur la tige fémorale 2. Toutefois, dans un souci de meilleure visibilité, seuls les signaux correspondants aux deuxième, troisième, quatrième, cinquième, neuvième, treizième, dix-huitième et vingt quatrième impacts sont représentés sur la FIG 2. On distingue clairement pour chaque signal deux premiers pics d'amplitude maximale.

Chaque impact est porté au temps t = 0. Le premier pic d'amplitude maximale P1 apparaît quasi-instantanément (i.e. une milliseconde après) au temps t1. Les premiers pics P1 des différents signaux sont superposés en temps sur la FIG 2, l'abscisse t1 du premier pic P1 étant la même pour chaque impact. L'amplitude du premier pic P1 reflète la force d'impaction exercée lors de l'impact.

Le deuxième pic d'amplitude maximale apparaît quelques dixièmes de milliseconde à quelques millisecondes après le premier pic P1. Comme indiqué précédemment, seuls les signaux correspondants aux impacts de rang 2, 3, 4, 5, 9, 13, 18 et 24 sont représentés sur la FIG 2. Ces rangs sont indiqués au dessus des deuxièmes pics d'amplitude maximale des signaux correspondants. Le deuxième pic apparaît à l'instant t2. Sur la FIG 2, le deuxième pic est repéré et noté P2 uniquement pour le troisième et le cinquième impact (ou impacts de rang 3 et 5). De même, l'instant t2 n'est repéré que pour le troisième et le cinquième impact.

Comme illustré par la FIG 2, la durée (t2 - t1) entre les premier et deuxième pics P1, P2 diminue en fonction du rang de l'impact et les inventeurs ont mis en évidence le fait que cette durée (t2 - t1) était un indicateur IN1 fiable et pertinent pour refléter le niveau de contact entre la tige fémorale 2 et le fémur 4.

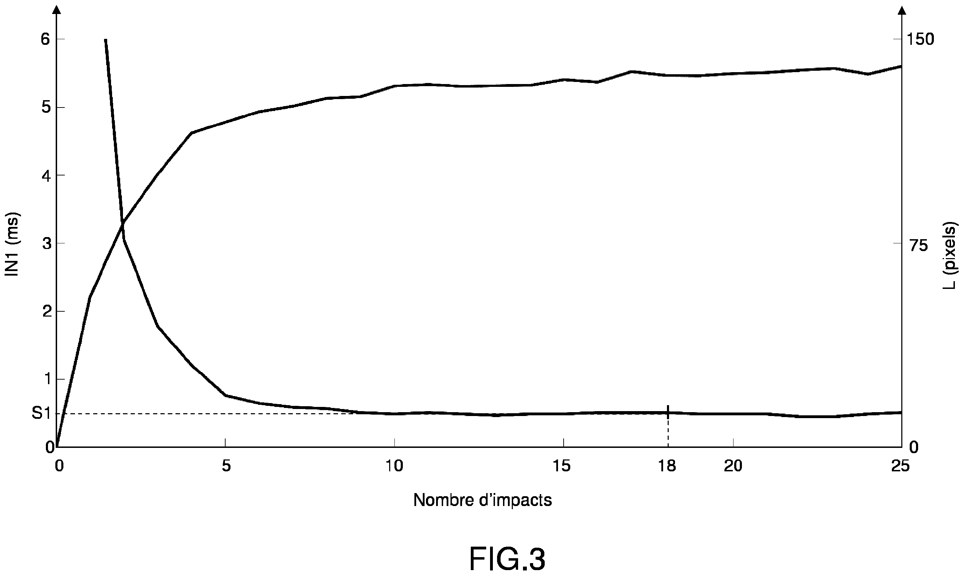

La FIG 3 est un graphique représentant l'évolution de l'indicateur IN1 (courbe en trait plein) en fonction du nombre d'impacts et l'évolution de la longueur d'enfoncement L de la tige 2 en fonction du nombre d'impacts lors du même test d'insertion que celui de la FIG. 2. L'insertion de la tige 2 dans le fémur 4 a été filmée et la longueur L a été mesurée sur les images vidéo (courbe en pointillés). Le nombre d'impacts (de 1 à 25) est reporté en abscisse. L'indicateur IN1, exprimé en millisecondes (ms), est reporté en ordonnée. La longueur de tige L enfoncée dans le fémur 4, mesurée sur les images vidéo et exprimée en nombre de pixels est également reportée en ordonnée.

Comme illustré par la FIG 3, de manière générale, plus la longueur d'enfoncement L de la tige fémorale 2 dans le fémur augmente, et donc plus la surface de la tige 2 au contact du fémur 4 augmente, plus l'indicateur IN1 diminue. Dans un premier temps, la longueur d'enfoncement L augmente rapidement et l'indicateur IN1 diminue rapidement. Ensuite, au bout d'un certain nombre d'impacts, la tige est arrivée "à quai" et ne s'enfonce pratiquement plus malgré les impacts. Ceci se traduit sur la FIG 3 par un palier dans l'évolution de la longueur d'enfoncement L. A ce moment, la surface de la tige 2 au contact du fémur 4 est maximum et ne varie pratiquement plus malgré les impacts. On constate qu'au même moment l'indicateur IN1 atteint lui aussi un palier. Ce graphique illustre le fait que l'indicateur IN1 est pertinent pour représenter le niveau de contact entre la tige 2 et le fémur 4.

En outre, dans ce test d'insertion, il a été considéré (notamment sur la base des images vidéo réalisées) que le niveau de contact tige-fémur était optimal ou, en tous cas, suffisant à partir du 18^{ème} impact. Sur cette base, la valeur de l'indicateur IN1 au 18^{ème} impact peut être choisie comme valeur seuil S1 pour paramétrer l'unité de traitement 30. Dans cet exemple, la valeur seuil S1 a été choisie égale à 0,32 ms. Bien entendu, il ne s'agit là que d'un exemple, et d'autres tests pourraient être réalisés, en alternative ou en combinaison, pour déterminer la valeur seuil S1 correspondant à un niveau de contact tige-fémur jugé suffisant. Typiquement, la valeur seuil S1 est comprise entre 0,1 et 1 ms.

Une fois déterminée, la valeur seuil S1 peut être utilisée pour paramétrer le dispositif d'insertion 10. Cette valeur seuil S1 est, par exemple, enregistrée dans la mémoire de l'unité de traitement 30. En outre, le dispositif 10 peut comprendre un système d'alerte 33 pour émettre un signal d'alerte (par exemple, un signal sonore, visuel et/ou tactile). Le système d'alerte 33 est relié à l'unité de traitement 30 et coopère avec celle-ci pour alerter le praticien lorsque le niveau de contact entre la tige 2 et le fémur 4 est jugé suffisant sur la base de l'indicateur IN1, c'est-à-dire, dans cet exemple, dès que l'indicateur IN1 descend en dessous de la valeur seuil S1. Dès lors, le praticien dispose en temps réel d'une information fiable lui indiquant qu'il a atteint un niveau de contact tige-fémur suffisant. Il en conclut qu'il peut arrêter d'impacter la tige 2, ce qui diminue le risque d'endommager le fémur 4, en particulier d'induire une fracture ou des microfissures dans le fémur 4.

L'exemple qui vient d'être décrit, concernant l'insertion d'une tige fémorale dans un fémur, est donné à titre illustratif et non limitatif, une personne du métier pouvant facilement tirer profit de l'indicateur proposé par les inventeurs avec d'autres types d'implants, sans sortir du cadre de l'invention. Autrement dit, la tige 2 et le fémur 4 ne sont, respectivement, que des exemples d'implant chirurgical et d'os récepteur au sens de l'invention.

En particulier, le dispositif proposé peut être utilisé pour des implants pour prothèse de hanche autres qu'une tige fémorale (e.g. pour des implants cotyloïdiens), des implants pour prothèse de genou, d'épaule, de rachis, de cheville, etc. et, plus généralement, tout type d'implant chirurgical nécessitant d'être inséré en force par impaction dans un os récepteur. Il peut également être utilisé pour l'insertion d'instruments chirurgicaux insérés temporairement dans le corps d'un patient et, par exemple, pour l'insertion d'une râpe chirurgicale comme une râpe fémorale pour prothèse de hanche. Les râpes fémorales sont prévues pour être insérées en force par impaction dans le canal médullaire afin de préparer ce canal à recevoir la tige fémorale. Ces râpes sont impactées directement par un impacteur, avec ou sans l'intermédiaire d'un ancillaire de pose. Si aucun ancillaire de pose n'est utilisé, la surface d'impact est alors constituée par une surface située au niveau de l'extrémité arrière d'une partie de préhension de la râpe.

En outre, l'exemple qui vient d'être décrit met en œuvre un capteur de force 12. Selon un autre exemple (non représenté), il est possible d'utiliser un capteur de déformation apte à fournir un signal de mesure représentant la variation temporelle de la déformation de l'impacteur 10 lors d'un impact. Dans une situation d'insertion d'une tige fémorale 2 comme celle représentée sur la FIG 1, un tel capteur de déformation est apte à convertir en un signal électrique exploitable la déformation de la tête de frappe 11 de l'impacteur 10. Dans ce cas, au lieu d'être situé sur la face de frappe 11a comme le capteur de force 12, le capteur de déformation est positionné sur une des faces latérales 11c de la tête de frappe 11. En l'occurrence, le capteur de déformation est positionné sur la face latérale 11c s'étendant parallèlement à la direction de l'axe du manche 13. Plus précisément, vu de côté (comme sur la FIG 1), le capteur de déformation est fixé sur la partie avant de la face latérale 11c, entre la face de frappe 11a et l'axe du manche 13. L'avant et l'arrière sont définis ici par rapport au mouvement de frappe de l'impacteur 10. Le capteur de déformation est fixé sur la tête de frappe 11, par exemple par collage ou tout autre moyen de fixation approprié, de sorte que la déformation de la tête de frappe 11 provoque la déformation du capteur. Le capteur est, par exemple, un capteur à jauge comprenant un élément de mesure élastique dont la déformation est d'abord convertie en une variation de résistance électrique de la jauge, pour générer ensuite un signal de sortie électrique. En variante, il peut s'agir d'un capteur piézo-électrique reposant sur les propriétés piézo-électriques d'un matériau (e.g. du quartz ou des céramiques synthétiques) qui génère une charge électrique lorsqu'il se déforme.

Le signal de mesure fourni par un tel capteur de déformation et représentant la variation temporelle de la déformation de l'impacteur lors d'un impact présente également des premier et deuxième pics d'amplitude maximale. Le laps de temps séparant ces deux pics s'avère également être un indicateur fiable et pertinent pour évaluer le niveau de contact entre l'implant et l'os récepteur.

Enfin, les différentes caractéristiques des modes ou exemples de réalisation décrits dans le présent exposé peuvent être considérées isolément ou être combinées entre elles. Lorsqu'elles sont combinées, ces caractéristiques peuvent l'être comme décrit ci-dessus ou différemment, l'invention ne se limitant pas aux combinaisons spécifiques précédemment décrites. En particulier, sauf précision contraire ou incompatibilité technique, une caractéristique décrite en relation avec un mode ou exemple de réalisation peut être appliquée de manière analogue à un autre mode ou exemple de réalisation.

## Revendications

1. Dispositif pour insérer en force un implant chirurgical dans un os récepteur, par impaction, comprenant:
un impacteur (10) adapté pour impacter une surface d'impact (20a) couplée à l'implant et exercer une force d'impaction sur l'implant,
au moins un capteur (12) associé à l'impacteur (10), et une unité de traitement (30) reliée au capteur (12),
le capteur (12) étant apte à mesurer une grandeur parmi la force d'impaction exercée et la déformation de l'impacteur (10), et à fournir un signal de mesure représentant la variation temporelle de ladite grandeur lors d'un impact,
l'unité de traitement (30) étant configurée pour calculer, à partir de la variation temporelle de ladite grandeur lors de l'impact, un indicateur (IN1) représentatif du niveau de contact entre l'implant et l'os récepteur, **caractérisé en ce que**
l'indicateur (IN1) correspond à la durée d'une fenêtre temporelle, le début de cette fenêtre temporelle étant défini par rapport à un instant (t1) correspondant au premier pic (P1) d'amplitude maximale du signal de mesure et la fin de cette fenêtre temporelle étant définie par rapport à un instant (t2) correspondant au deuxième pic d'amplitude maximale (P2) du signal de mesure.

2. Dispositif selon la revendication 1 comprenant, en outre, un système d'alerte (33) relié à l'unité de traitement (30) et coopérant avec celle-ci de manière à émettre un signal d'alerte lorsque l'indicateur (IN1) devient inférieur à une valeur seuil (S1) prédéterminée.

3. Dispositif selon la revendication 2, dans lequel la valeur seuil (S1) prédéterminée est comprise entre 0,1 et 1 ms.

4. Dispositif selon l'une quelconque des revendications 1 à 3, dans lequel l'unité de traitement (30) est configurée pour détecter dans le signal de mesure le premier pic (P1) d'amplitude maximale et le pic d'amplitude maximale succédant au premier pic (P1), ce dernier pic n'étant considéré comme le deuxième pic d'amplitude maximale (P2) que si l'amplitude du signal devient inférieure à une valeur limite prédéterminée entre les deux pics.

5. Dispositif selon la revendication 4, dans lequel la valeur limite prédéterminée est comprise entre 1 et 20 % de l'amplitude maximale du premier pic (P1).

6. Dispositif selon l'une quelconque des revendications 1 à 5, dans lequel l'impacteur (10) a une face de frappe (11a) adaptée pour impacter la surface d'impact (20a), une face opposée (11b) à la face de frappe et des faces latérales (11c) s'étendant entre la face de frappe (11a) et la face opposée (11b), et dans lequel le capteur est un capteur de déformation (12) apte à mesurer la déformation de l'impacteur (10) et à fournir un signal de mesure représentant la variation temporelle de la déformation de l'impacteur (10) lors d'un impact.

7. Dispositif selon l'une quelconque des revendications 1 à 6, dans lequel l'impacteur (10) a une face de frappe (11a) adaptée pour impacter la surface d'impact (20a) et dans lequel le capteur est un capteur de force apte à mesurer la force d'impaction et à fournir un signal de mesure représentant la variation temporelle de la force d'impaction lors d'un impact.

8. Dispositif selon l'une quelconque des revendications 1 à 7, dans lequel l'impacteur (10) est un marteau, ou équivalent, et comprend un manche (13) surmonté d'une tête de frappe (11).

9. Dispositif selon l'une quelconque des revendications 1 à 8, comprenant, en outre, un ancillaire de pose (20) ayant une extrémité arrière formant ladite surface d'impact (20a) et une extrémité avant (20b) adaptée pour coopérer avec l'implant, l'impacteur (10) exerçant la force d'impaction sur l'implant par l'intermédiaire de l'ancillaire de pose (20).

10. Ensemble comprenant un dispositif selon l'une quelconque des revendications 1 à 9 et un implant chirurgical, en particulier une tige fémorale (2).

11. Ensemble selon la revendication 10 comprenant un dispositif selon la revendication 9, dans lequel l'extrémité avant (20b) de l'ancillaire de pose est attachée mécaniquement à l'implant de manière amovible, par exemple par vissage.

## Patentansprüche

1. Vorrichtung zum mit Kraft erfolgenden Einsetzen eines chirurgischen Implantats in einen aufnehmenden Knochen durch Einschlagen, mit:
einen Impaktor (10), der dazu ausgebildet ist, auf eine mit dem Implantat gekoppelte Aufschlagfläche (20a) zu schlagen und eine Einschlagkraft auf das Implantat auszuüben,
mindestens einem dem Impaktor (10) zugeordneten Sensor (12), und
einer mit dem Sensor (12) verbundene Verarbeitungseinheit (30),
wobei der Sensor (12) dazu ausgebildet ist, unter der ausgeübten Einschlagkraft und der Verformung des Impaktors (10) eine Größe zu messen und ein Messsignal zu liefern, das die zeitliche Änderung der genannten Größe während eines Aufschlags darstellt,
wobei die Verarbeitungseinheit (30) dazu ausgebildet ist, aus der zeitlichen Änderung der Größe während des Aufschlags einen Indikator (IN1) zu berechnen, der für das Kontaktniveau zwischen dem Implantat und dem aufnehmenden Knochen repräsentativ ist,
**dadurch gekennzeichnet, dass**
der Indikator (IN1) der Dauer eines Zeitfensters entspricht, wobei der Beginn dieses Zeitfensters in Bezug auf einen Zeitpunkt (t1) definiert ist, welcher der ersten maximalen Amplitudenspitze (P1) des Messsignals entspricht, und wobei das Ende des Zeitfensters in Bezug auf einen Zeitpunkt (t2) definiert ist, welcher der zweiten maximalen Amplitudenspitze (P2) des Messsignals entspricht.

2. Vorrichtung nach Anspruch 1, ferner mit einem Warnsystem (33), das mit der Verarbeitungseinheit (30) verbunden ist und mit dieser zusammenwirkt, um ein Alarmsignal auszugeben, wenn der Indikator (IN1) einen vorbestimmten Schwellwert (S1) unterschreitet.

3. Vorrichtung nach Anspruch 2, bei welcher der vorbestimmte Schwellenwert (S1) zwischen 0,1 und 1 ms beträgt.

4. Vorrichtung nach einem der Ansprüche 1 bis 3, bei welcher die Verarbeitungseinheit (30) dazu ausgebildet ist, in dem Messsignal die erste maximale Amplitudenspitze (P1) und die auf die erste Spitze (P1) folgende Spitze der maximalen Amplitudenspitze zu detektieren, wobei die letztere Spitze nur dann als die zweite maximale Amplitudenspitze (P2) angesehen wird, wenn die Amplitude des Signals einen vorbestimmten Grenzwert zwischen den beiden Spitzen unterschreitet.

5. Vorrichtung nach Anspruch 4, bei welcher der vorgegebene Grenzwert zwischen 1 und 20 % der maximalen Amplitude der ersten Spitze (P1) beträgt.

6. Vorrichtung nach einem der Ansprüche 1 bis 5, bei welcher der Impaktor (10) eine Schlagfläche (11a) aufweist, die dazu ausgebildet ist, auf die Aufschlagfläche (20a) zu schlagen, eine der Schlagfläche gegenüberliegende Fläche (11b) und Seitenflächen (11c) aufweist, die sich zwischen der Schlagfläche (11a) und der gegenüberliegenden Fläche (11b) erstrecken, und wobei der Sensor ein Verformungssensor (12) darstellt, der dazu ausgebildet ist, die Verformung des Impaktors (10) zu messen und ein Messsignal zu liefern, das die zeitliche Änderung der Verformung des Impaktors (10) während eines Aufschlags darstellt.

7. Vorrichtung nach einem der Ansprüche 1 bis 6, bei welcher der Impaktor (10) eine Schlagfläche (11a) aufweist, die dazu ausgebildet ist, auf die Aufschlagfläche (20a) zu schlagen und wobei der Sensor ein Kraftsensor ist, der dazu ausgebildet ist, die Aufschlagkraft zu messen und ein Messsignal, zu liefern, das die zeitliche Variation der Aufschlagkraft während eines Aufschlags darstellt.

8. Vorrichtung nach einem der Ansprüche 1 bis 7, wobei der Impaktor (10) ein Hammer oder dergleichen ist und einen Griff (13) aufweist, auf welchem ein Schlagkopf (11) angebracht ist.

9. Vorrichtung nach einem der Ansprüche 1 bis 8, ferner mit einer Positionierungshilfseinrichtung (20) mit einem hinteren Ende, welches die Aufschlagfläche (20a) bildet, und einem vorderes Ende (20b), das dazu ausgebildet ist, mit dem Implantat zusammenzuwirken, wobei der Impaktor (10), die Schlagkraft auf das Implantat über die Positionierungshilfseinrichtung (20)
ausübt.

10. Anordnung mit einer Vorrichtung nach einem der Ansprüche 1 bis 9 und einem chirurgischen Implantat, insbesondere einem Oberschenkelschaft (2).

11. Anordnung nach Anspruch 10, mit einer Vorrichtung nach Anspruch 9, bei welcher das vordere Ende (20b) der die Positionierungshilfseinrichtung mechanisch, beispielsweise durch Verschrauben, am Implantat lösbar befestigt ist.

## Claims

1. A device for forcibly inserting a surgical implant into a receiving bone, by impaction, comprising:
an impactor (10) adapted for impacting an impact surface (20a) coupled to the implant and exerting an impact force on the implant,
at least one sensor (12) associated with the impactor (10), and
a processing unit (30) connected to the sensor (12),
the sensor (12) being capable of measuring a magnitude out of the impact force exerted and the deformation of the impactor (10), and of providing a measurement signal representing the temporal variation of said magnitude during an impact,
the processing unit (30) being configured to calculate, on the basis of the temporal variation of said magnitude during the impact, an indicator (IN1) representative of the level of contact between the implant and the receiving bone, **characterized in that**
the indicator (IN1) corresponds to the duration of a time window, the start of the time window being defined with respect to an instant (t1) corresponding to the first peak (P1) of maximum amplitude of the measurement signal and the end of the time window being defined with respect to an instant (t2) corresponding to the second peak of maximum amplitude (P2) of the measurement signal.

2. The device according to claim 1, further comprising an alert system (33) connected to the processing unit (30) and interacting with the latter so as to emit an alert signal when the indicator (IN1) becomes less than a predetermined threshold value (S1).

3. The device according to claim 2, wherein the predetermined threshold value (S1) is between 0.1 and 1 ms.

4. The device according to any one of claims 1 to 3, wherein the processing unit (30) is configured to detect, in the measurement signal, the first peak (P1) of maximum amplitude and the peak of maximum amplitude following the first peak (PI), the latter peak being considered as the second peak of maximum amplitude (P2) only if the amplitude of the signal between the two peaks becomes less than a predetermined limit value.

5. The device according to claim 4, wherein the predetermined limit value is between 1 and 20% of the maximum amplitude of the first peak (P1).

6. The device according to any one of claims 1 to 5, wherein the impactor (10) has a striking face (11a) for impacting the impact surface (20a), an opposite face (11b), opposite to the striking face, and side faces (11c) extending between the striking face (11a) and the opposite face (11b), and wherein the sensor is a deformation sensor (12) adapted for measuring a deformation of the impactor (10) and providing a measurement signal representing the temporal variation of the deformation of the impactor (10) during an impact.

7. The device according to any one of claims 1 to 6, wherein the impactor (10) has a striking face (11a) for impacting the impact surface (20a) and wherein the sensor is a force sensor adapted for measuring the impact force and providing a measurement signal representing the temporal variation of the impact force during an impact.

8. The device according to any one of claims 1 to 7, wherein the impactor (10) is a hammer, or equivalent, and comprises a gripping shaft (13) topped by a striking head (11).

9. The device according to any one of claims 1 to 8, further comprising an ancillary tool (20) having a rear end forming said impact surface (20a) and a front end (20b) adapted for cooperating with the implant, the impactor (10) exerting the impact force on the implant via the ancillary tool (20).

10. An assembly comprising a device according to any one of claims 1 to 9 and a surgical implant, in particular a femoral stem (2).

11. The assembly according to claim 10, comprising a device according to claim 9, wherein the front end (20b) of the ancillary tool is mechanically attached to the implant in a removable manner, for example by screwing.
